# EUROPÄISCHE PATENTSCHRIFT

(11) **EP 3 294 156 B1**
(45) Veröffentlichungstag und Bekanntmachung des Hinweises auf die Patenterteilung: **26.12.2018**
(21) Anmeldenummer: 16750090.9
(22) Anmeldetag: 06.05.2016
(51) Int. Cl.: A61B 17/16, A61C 8/00, A61B 10/02

(54) **CHIRURGISCHES HANDGERÄT SOWIE EINE SCHUTZEINRICHTUNG**
SURGICAL HAND-HELD INSTRUMENT AND A PROTECTION DEVICE
APPAREIL À MAIN CHIRURGICAL ET UNE DISPOSITIF DE PROTECTION

(30) Priorität: 08.05.2015 DE 102015208609; 11.05.2015 DE 102015208646
(43) Veröffentlichungstag der Anmeldung: 21.03.2018
(73) Patentinhaber: Zastrow, Frank, 69120 Heidelberg (DE)
(72) Erfinder: Zastrow, Frank, 69120 Heidelberg (DE)
(74) Vertreter: Patent- und Rechtsanwälte Ullrich & Naumann
(86) Internationale Anmeldenummer: PCT/DE2016/200216
(87) Internationale Veröffentlichungsnummer: WO 2016/180416

(56) Entgegenhaltungen:
- WO-A1-2009/005458
- DE-A1-102007 060 493
- DE-A1-102008 019 790
- DE-A1-102011 012 637

## Beschreibung

Die Erfindung betrifft ein chirurgisches Handgerät, nämlich Handstück oder Winkelstück zur Anwendung in der Oralchirurgie, mit einem rotierbaren Werkzeug, wobei ein Kopf des Werkzeugs als Hohlzylinder ausgebildet ist und wobei an einem distalen Rand des Kopfes ein Wirkbereich, insbesondere zur spanenden Bearbeitung von Knochen, angeordnet ist. Des Weiteren betrifft die Erfindung eine Kombination aus einem rotierbaren Werkzeug und einer Schutzeinrichtung zur Anwendung in der Oralchirurgie.

Chirurgisch tätige Zahnärzte, Oralchirurgen und Kieferchirurgen stehen häufig vor der Herausforderung, dass durch Knochenatrophie, durch Unfälle, durch Parodontitis oder durch Zahnextraktion Knochen in der Mundhöhle verloren gegangen ist.

Wenn Zahnimplantate zum Einsatz neuer Zähne geplant werden, so ist es wichtig, dass diese Knochendefizite vorher oder simultan mit der Implantatsetzung aufgebaut werden, damit die Zahnimplantate wieder ein neues Fundament und eine stabile Abstützung im Knochen haben.

Autologer, patienteneigener Knochen gilt dabei nach wie vor als Goldstandard bei knochenaufbauenden Eingriffen. Dies liegt an den Eigenschaften des Knochens, da autologer Knochen sowohl osteogene, osteoinduktive sowie osteokonduktive Eigenschaften miteinander vereint. Das bedeutet, der Knochen hat die Potenz, eigenes Knochengewebe zu bilden, Gefäße zu bilden und wirkt zudem als Leitstruktur für neu gebildeten Knochen. Knochenersatzmaterial hat im Gegensatz zu autologem Knochen keine biologische Potenz und wirkt lediglich osteokonduktiv, das heißt, es wirkt auch als Leitschiene.

Beim Arbeiten mit autologem Knochen sind verschiedene Verfahren vorbekannt. Bei größeren Knochendefiziten bieten sich als zweite intraorale Entnahmestelle grundsätzlich zahnlose Knochenareale an, alternativ der Tuber maxillae, die Spina nasalis anterior, der Gaumen, der Bereich der Kieferhöhlenwand im Oberkiefer oder aber der Unterkiefer, da dieser eher kortikaler Natur ist und die Knochenqualität als sehr gut und stabil gilt. Im Unterkiefer gibt es verschiedene Knochenentnahmestellen, so beispielsweise wiederum zahnlose Areale, das Kinn oder den retromolare Bereich.

Aus der US 2,429,356 ist ein Knochenschleifinstrument mit einem Werkzeug vorbekannt, das als kugelförmigen Bohrkopf bzw. als Fräse in Form eines abgerundeten Kegels ausgebildet ist. Das Werkzeug ist zumindest teilweise von einer Schutzeinrichtung umgeben.

Die DE 10 2009 013 451 A1 betrifft ein chirurgisches Instrument zum Abtragen von Knochen. Das Instrument weist einen Bohrkopf auf, der von einer Schutzeinrichtung teilweise umgeben ist.

Das Dokument DE 32 02 193 A1 offenbart ein hohlzylinderförmiges Werkzeug zur Entnahme von Knochenzylindern.

Die Knochenentnahme kann hierbei mit verschiedenen Instrumenten erfolgen. Das Konzept der Knochenentnahme ist dabei meist ähnlich.

Entweder werden mit einer sogenannten Lindemannfräse oder mit einem Piezosurgerygerät oder aber mit einer kleinen Säge drei bis vier Sollbruchstellen geschaffen und der Block im Nachhinein mit einem Meisel oder einem anderen Instrument herausgebrochen. Dabei ist nachteilig, dass bei einer Knochenentnahme mehr oder weniger große Gewaltanwendung auf den Kiefer nötig ist. Daher wird dieser Eingriff teilweise vom Arzt gescheut, auch aus dem Grund, da dieses Heraushämmern bzw. -brechen des Knochenblockes aus der jeweiligen Region auch für den Patienten unangenehm ist.

Zahnärzte sind aufgrund Ihres Berufes rotierende Instrumente und Bohrer gewohnt. So hat sich auch die sogenannte Trepanfräse etabliert, die an ein Handstück angesteckt wird und einen Kopf aufweist, der als Hohlzylinder ausgebildet ist. An dem distalen Rand des Kopfes sind Zähne zur spanenden Bearbeitung des Knochens ausgebildet. Trepanfräsen werden beispielsweise zur Implantatbettaufbereitung genutzt und weisen einen Durchmesser von ca. 3 mm bis 4 mm auf. Mit Hilfe dieser Fräsen werden äußerst kleine und schmale Bohrzylinder entnommen, die nur bedingt zum Knochenaufbau geeignet sind.

Bei den voranstehend genannten Vorrichtungen müssen zur Knochenentnahme ca. drei bis vier Schnitte bzw. Sollbruchstellen angelegt werden, um das benötigte Knochenstück zu erhalten. Dies bedingt eine starke Belastung für den Patienten und erfordert großes handwerkliches Geschick durch den Operateur. Insbesondere ist dabei zu beachten, dass das umliegende Weichgebewebe, beispielsweise Wange oder Lippe, durch das chirurgische Werkzeug nicht verletzt wird.

Der vorliegenden Erfindung liegt daher die Aufgabe zugrunde, ein chirurgisches Handgerät der eingangs genannten Art derart auszugestalten und weiterzubilden, dass mit konstruktiv einfachen Mitteln eine zuverlässige und für den Patienten schonende Knochenentnahme möglich ist.

Erfindungsgemäß wird die voranstehende Aufgabe durch die Merkmale des Anspruches 1 gelöst. Danach ist das in Rede stehende chirurgische Handgerät dadurch gekennzeichnet, dass eine Schutzeinrichtung angeordnet ist, die den distalen Rand des Werkzeugs teilweise umgibt und sich in Axialrichtung teilweise über den distalen Rand hinweg erstreckt, so dass lediglich ein Kreisbogen des distalen Randes als Wirkbereich dient.

In erfindungsgemäßer Weise ist zunächst erkannt worden, dass sich entgegen eines Vorurteils der Fachwelt ein Werkzeug mit einem als Hohlzylinder ausgebildeten Kopf, der einen Wirkbereich zur spanenden Bearbeitung von Knochen aufweist, nicht nur zur Entnahme von kleinen Bohrzylindern eignet. Vielmehr lässt sich ein entsprechend ausgebildetes Werkzeug dazu nutzen, das benötigte Knochenstück bzw. Knochensegment aus dem Knochen "herauszuschälen". In weiter erfindungsgemäßer Weise ist dabei erkannt worden, dass durch die Anordnung einer Schutzeinrichtung, die den Kopf des Werkzeugs, insbesondere radial bzw. in Umfangsrichtung, teilweise umgibt und sich in Axialrichtung teilweise über den distalen Rand hinweg erstreckt, lediglich ein Kreisbogen des distalen Randes des Kopfes mit dem Knochen in Kontakt bringbar ist und somit als effektiver Wirkbereich dient. Durch diese konstruktive Maßnahme ist es dem Operateur möglich, ein etwa halbmondförmiges Knochenstück aus dem Kochen abzuschälen, das für den Knochenaufbau verwendbar ist. Im Gegensatz zu den aus dem Stand der Technik bekannten Instrumenten bzw. Werkzeugen müssen keine drei bis vier Schnitte bzw. Sollbruchstellen angelegt werden. Es entsteht vielmehr lediglich eine einzige Sollbruchstelle, ein Heraushämmern des Knochens ist nicht notwendig. Der entstehende Block ist sodann ohne größere Gewalteinwirkung herauslösbar bzw. luxierbar, was für den Patienten im Vergleich zu den bekannten Vorrichtungen und Techniken schonender und angenehmer ist. Folglich ist die Belastung durch den Eingriff für den Patienten minimal. Des Weiteren ist das umliegende Gewebe durch die Schutzeinrichtung in idealer Weise vor Verletzungen geschützt. Somit ist das chirurgische Handgerät besonders einfach in der Handhabung.

In vorteilhafter Weise umgibt die Schutzeinrichtung den Kopf derart, dass ein von dem Kreisbogen gebildetes Kreissegment eine Segmenthöhe von 2,5 mm bis 3,5 mm, insbesondere 2,7 mm bis 3,3 mm, vorzugsweise 2,9 mm aufweist. Im Konkreten kann die Segmenthöhe über eine Einstellschraube einstellbar sein, wobei die Schutzeinrichtung über die Einstellschraube radial gegenüber dem Werkzeug verschiebbar ist. Dadurch ist sicher gestellt, dass das Werkzeug nur so weit in den Knochen eindringt, dass eine Verletzung des in dem Knocheninneren verlaufenden Nervs vermieden wird. Dabei kann die Schutzhülle des Weiteren derart dimensioniert sein, dass der Kopf in Axialrichtung maximal 12 mm bis 22 mm, insbesondere 14 mm bis 18 mm, vorzugsweise 15 mm, tief in den Knochen einbringbar ist, um eine Verletzung der innenliegenden Nerven zu verhindern

Damit das entnommene Knochenstück zur Rekonstruktion eines Implantatlagers geeignet ist, kann die Schutzeinrichtung weiterhin derart dimensioniert sein, dass die Kreissehne des Kreissegments eine Länge von 8 mm bis 15 mm aufweist.

In weiter vorteilhafter Weise ist/sind die Schutzeinrichtung und/oder das Werkzeug lösbar mit dem Handgerät verbunden. Bei dem Handgerät kann es sich dabei um ein gängiges Winkelstück oder Handstück handeln, wie es von Zahnärzten bzw. Dentalchirurgen verwendet wird. Die Schutzeinrichtung kann an das Handgerät über eine Steckverbindung, eine Schraubverbindung oder eine Bajonettverbindung fixierbar sein. Ferner kann die Schutzeinrichtung in Umfangsrichtung drehbar ausgebildet sein, um eine Anpassung an die Entnahmestelle des Knochens zu ermöglichen. Im Konkreten kann das Werkzeug in das Handgerät einspannbar sein. Alternativ oder zusätzlich kann das Werkzeug, insbesondere dessen Anschlussbereich bzw. Schaft, drehbar mit der Schutzeinrichtung verbunden bzw. gekoppelt sein. Zwischen der Schutzeinrichtung und dem Werkzeug kann dazu ein Lager, insbesondere ein Wälzlager bzw. Kugellager bzw. Rillenkugellager, ausgebildet sein. Mit dem Verbinden des Anschlussbereiches des Werkzeugs - beispielsweise einem üblicherweise bei solchen Werkzeugen angeordneten Schaft - mit dem chirurgischen Handgerät ist auch die Schutzeinrichtung mit dem chirurgischen Handgerät verbunden. Somit dient der Anschlussbereich des Werkzeugs auch als Verbindungselement der Schutzeinrichtung, um nämlich eine, vorzugsweise lösbare, Verbindung mit dem chirurgischen Handgerät zu realisieren.

Wie bereits voranstehend ausgeführt ist, kann das Werkzeug einen Anschlussbereich zum Verbinden mit einem Handgerät, bspw. einem zahnärztlichen Winkelstück oder Handstück aufweisen. Des Weiteren können an dem distalen Rand des Kopfes eine raue Oberfläche und/oder Zähne, insbesondere Schneidzähne bzw. Sägezähne, ausgebildet sein. Die raue Oberfläche und/oder die Zähne können zumindest einen Teil des Wirkbereichs bilden.

Um ein Verkanten und dadurch bedingtes Abrutschen zu verhindern bzw. ein "ruckelfreies", weiches Eindringen des Werkzeugs in den Knochen zu gewährleisten, kann zumindest an der an den distalen Rand angrenzenden inneren und/oder äußeren Wandung des Kopfes eine raue Oberfläche ausgebildet sein, die einen Teil des Wirkbereichs bildet. Dadurch ist es dem Operateur möglich, eine Richtungsänderung der Bewegung des Werkzeugs innerhalb des Knochens vorzunehmen.

In vorteilhafter Weise ist die raue Oberfläche mechanisch, elektroerosiv oder durch Ätzen erzeugt. Des Weiteren ist denkbar, dass die raue Oberfläche durch eine Beschichtung mit Diamantkörner oder Korrundkörnern realisiert ist. Im Konkreten kann es sich bei dem Werkzeug um ein Werkzeug aus Metall, beispielsweise Edelstahl handeln, wobei die raue Oberfläche mechanisch, elektroerosiv, durch Ätzen oder durch eine Beschichtung mit Diamantkörnern oder Korrundkörnern realisiert ist. Insbesondere Diamantkörner bieten aufgrund ihrer Härte die Möglichkeit einer besonders schonenden und schnellen Bearbeitung des Knochens.

Zur Vereinfachung der Handhabung des Werkzeugs kann an der äußeren Wandung des Kopfes eine Markierung zur Visualisierung der Eindringtiefe des Kopfes in den Knochen ausgebildet sein. Dies bietet dem Operateur eine einfache Möglichkeit, die Eindringtiefe zu kontrollieren und somit die Verletzung von in dem Knochen liegenden Nerven zu vermeiden. Dabei ist des Weiteren denkbar, dass an dem Kopf eine Eindringsperre ausgebildet ist, um ein zu tiefes Eindringen des Werkzeugs in den Knochen zu vermeiden. Diese konstruktive Maßnahme dient ebenfalls dem Schutz der innerhalb des Knochens verlaufenden Nerven. Insbesondere kann die Eindringsperre als an der inneren und/oder äußeren Wandung des Kopfes angeordneter Vorsprung realisiert sein. Zur Beaufschlagung des Werkzeugs und/oder der Entnahmestelle mit einem Kühlmedium - bspw. einer NaCI-Lösung - kann an dem Werkzeug mindestens eine Austrittsöffnung ausgebildet sein, wobei die Austrittsöffnung mit einem in dem Werkzeug verlaufenden Kanal in Strömungsverbindung steht.

Eine besonders schonende Bearbeitung des Knochens ist möglich, wenn die Wandung des Kopfes eine Dicke von 0,1 mm bis 1, 5 mm, insbesondere von 0,4 mm bis 0,8 mm aufweist. Als besonders vorteilhaft hat sich dabei eine Dicke von 0,5 mm erwiesen, bei der die Wandung des Kopfes die benötigte Stabilität aufweist und dabei eine möglichst dünne Ausgestaltung realisiert ist, welche den Knochen schonend zu bearbeiten.

Des Weiteren ist denkbar, dass der distale Rand des Kopfes einen konvexen, insbesondere runden oder ovalen, Querschnitt aufweist. Eine solche Geometrie ermöglicht ein besonders "weiches" Eindringen des Werkzeugs in den Knochen. Alternativ kann der distale Rand des Kopfes einen eckigen, insbesondere dreieckigen, Querschnitt aufweisen, wodurch auch extrem harte Knochenschichten sehr schonend bearbeitet werden können.

In weiter vorteilhafter Weise kann der Innendurchmesser des Kopfes 5 mm bis 20 mm, beispielsweise 5 mm bis 10 mm, insbesondere 5 mm bis 8 mm, vorzugsweise 5 mm bis 6 mm, betragen. Ein entsprechend großer Kopf bietet somit die Möglichkeit, dass sich Knochenstücke mit der benötigten Dimensionierung aus dem Knochen entnehmen lassen. Alternativ oder zusätzlich kann der Kopf in axialer Richtung eine Länge von 8 mm bis 23 mm, insbesondere 10 mm bis 18 mm, vorzugsweise 15 mm aufweisen. Auch durch dieses Merkmal ist gewährleistet, dass das zu entnehmende Knochenstück die benötigte Größe aufweist, um als Fundament zur Implantatsetzung zu dienen.

Die Wandung des Kopfes kann zumindest im Bereich der rauen Oberfläche durchgehend massiv ausgebildet sein, so dass die den Wirkbereich bildende raue Oberfläche maximal ist. Ferner kann die Wandung des Kopfes zumindest im Bereich der rauen Oberfläche oder über den gesamten hohlzylinderförmigen Bereich hinweg Ausnehmungen aufweisen. Die Ausnehmungen können dabei beispielsweise oval ausgebildet sein. Durch die Ausnehmungen wird eine Überhitzung der Entnahmestelle bzw. des Werkzeugs vermieden.

Um eine möglichst klein dimensionierte Schutzeinrichtung zu realisieren, die umliegendes Weichgewebe vor dem rotierenden Werkzeug schützt, kann die Schutzeinrichtung zumindest bereichsweise zylinderförmig ausgebildet sein. In idealer Weise ist die Schutzeinrichtung im Bereich des hohlzylinderförmigen Kopfes zylinderförmig bzw. zylindersegmentförmig ausgebildet.

In weiter vorteilhafter Weise kann die Schutzeinrichtung zumindest aus zwei Teilen bestehen, wobei die Teile mit einem Klebstoff miteinander verbunden sein können. Um eine Wiederaufbereitung und damit einhergehende Probleme bzgl. der Hygiene zu vermeiden, kann der Klebstoff derart ausgewählt bzw. eingestellt sein, dass er bei einer Wiederaufbereitung bzw. Reinigung, bspw. Autoklavierung, schmilzt, so dass die Schutzeinrichtung nicht mehr verwendbar ist. Alternativ oder zusätzlich kann die Schutzeinrichtung aus einem Material gefertigt sein, dass bei einer Wiederaufbereitung seine Form und/oder Farbe ändert.

Zur weiteren Verbesserung der Handhabbarkeit und zur Vermeidung von Verletzungen des umliegenden Gewebes kann an dem freien Ende der Schutzeinrichtung ein sich radial nach innen erstreckender Vorsprung ausgebildet sein. Dadurch ist der distale Rand des Kopfes nicht nur in Umfangsrichtung von der Schutzeinrichtung umgeben, sondern auch radial bzw. axial. Der Vorsprung kann dabei derart ausgebildet sein, dass der distale Rand des Kopfes von einer Art Kragen umschlossen ist. Somit kann ein Teil des Vorsprungs auch radial innerhalb des Kopfes verlaufen.

Weiterhin kann an dem freien Ende der Schutzeinrichtung ein radial über das freie Ende hinweg verlaufender, vorzugsweise runder, Steg ausgebildet sein. Durch den Steg ist ein Tiefenschutz realisiert, der ein zu tiefes Eindringen des Werkzeugs in den Knochen verhindert. Alternativ kann die Schutzeinrichtung am freien Ende geschlossen sein.

In besonders vorteilhafter Weise kann an der Schutzeinrichtung zumindest eine Austrittöffnung angeordnet sein, wobei die Austrittsöffnung mit einem in der Schutzeinrichtung verlaufenden Kanal in Strömungsverbindung steht, um das Werkzeug, den Wirkbereich und/oder die Eingriffsstelle mit einem Kühlmedium zu beaufschlagen. Dabei ist denkbar, dass die Austrittsöffnung mit mehreren Kanälen in Strömungsverbindung steht und/oder dass mehrere Austrittsöffnungen vorgesehen sind. Bei dem Kühlmedium kann es sich beispielsweise um eine NaCI-Lösung handeln. Des Weiteren kann die Austrittsöffnung derart als Düse ausgebildet sein, dass das Kühlmedium weitflächig ausgetragen wird oder nahezu punktuell austritt.

Um dem Operateur eine gute Sicht auf die Eingriffsstelle zu ermöglichen, kann die Schutzeinrichtung in Umfangsrichtung nebeneinander angeordnete Öffnungen aufweisen. Im Konkreten können die Öffnungen oval ausgebildet sein. Alternativ oder zusätzlich kann die Schutzeinrichtung aus einem durchsichtigen Material, vorzugweise Kunststoff, hergestellt sein. Zur Vereinfachung der Handhabung ist es möglich, an der äußeren Wandung der Schutzeinrichtung Tiefenmarkierungen anzubringen.

In ganz besonders vorteilhafter Weise kann an der Schutzeinrichtung ein sich in Axialrichtung erstreckendes Führungselement angeordnet sein. Das Führungselement dient zum leichteren Einführen der Vorrichtung in die "Tasche" zwischen Knochen und Weichgewebe. Das Führungselement kann an dem von dem Handgriff entfernten Ende gebogen ausgebildet sein, um ein besonders leichtes Einbringen in die "Tasche" zu gewährleisten.

Die zugrundeliegende Aufgabe ist des Weiteren durch eine Kombination aus einem rotierbaren Werkzeug und einer Schutzeinrichtung nach Anspruch 12 gelöst. Danach ist eine Kombination aus einem rotierbaren Werkzeug und einer Schutzeinrichtung, zur Anwendung in der Oralchirurgie, wobei das Werkzeug einen mit einem chirurgischen Handgerät verbindbaren Anschlussbereich aufweist, der vorgesehen ist, für die Schutzeinrichtung als Verbindungselement zur lösbaren Befestigung an dem chirurgischen Handgerät zu dienen, wobei die Schutzeinrichtung drehbar mit dem Werkzeug gekoppelt ist und von dem Werkzeug getragen wird, wobei ein Kopf des Werkzeugs hohlzylinderförmig ausgebildet ist, wobei die Schutzeinrichtung einen distalen Rand des Werkzeugs teilweise umgibt und sich in Axialrichtung teilweise über den distalen Rand hinweg erstreckt, so dass lediglich ein Kreisbogen des distalen Randes als Wirkbereich, insbesondere zur spanenden Bearbeitung von Knochen, dient, beansprucht.

Die Schutzeinrichtung und/oder das Werkzeug kann gemäß der in den Ansprüchen 1 bis 11 beschriebenen Schutzeinrichtung bzw. dem dort beschriebenen Werkzeug ausgestaltet sein und die voranstehend in Bezug auf diese Schutzeinrichtung bzw. dieses Werkzeug genannten Merkmale und Vorteile aufweisen. Des Weiteren kann die Schutzeinrichtung gemäß Anspruch 12 sowie das Werkzeug sämtliche Merkmale der in der nachfolgenden Figurenbeschreibung beschriebenen Schutzeinrichtung bzw. des beschriebenen Werkzeugs aufweisen.

In erfindungsgemäßer Weise ist die Schutzeinrichtung drehbar mit dem Werkzeug verbunden bzw. gekoppelt, so dass die Schutzeinrichtung von dem Werkzeug, insbesondere dem Schaft bzw. Anschlussbereich, getragen wird. Insbesondere kann die Kopplung bzw. Verbindung über ein Lager, beispielsweise ein Wälzlager bzw. Kugellager bzw. Rillenkugellager erfolgen. Bei einer solchen Ausgestaltung dient ein Schaft bzw. Anschlussbereich des Werkzeugs als Verbindungselement für die Schutzeinrichtung, um diese mit einem chirurgischen Handgerät lösbar zu verbinden. Somit ist mit konstruktiv einfachen Mitteln eine Kopplung der Schutzeinrichtung mit einem chirurgischen Handgerät möglich.

Es gibt nun verschiedene Möglichkeiten, die Lehre der vorliegenden Erfindung in vorteilhafter Weise auszugestalten und weiterzubilden. Dazu ist einerseits auf die den Ansprüchen 1 und 12 nachgeordneten Ansprüche und andererseits auf die nachfolgende Erläuterung bevorzugter Ausführungsbeispiele der Erfindung anhand der Zeichnung zu verweisen. In Verbindung mit der Erläuterung der bevorzugten Ausführungsbeispiele der Erfindung anhand der Zeichnung werden auch im Allgemeinen bevorzugte Ausgestaltungen und Weiterbildungen der Lehre erläutert. In der Zeichnung zeigen
- Fig. 1: in einer schematischen, perspektivischen Darstellung ein Ausführungsbeispiel einer Schutzeinrichtung sowie eines Werkzeugs eines erfindungsgemäßen chirurgischen Handgeräts,
- Fig. 2: in einer schematischen, frontalen Darstellung das Ausführungsbeispiel gemäß Fig. 1,
- Fig. 3: in einer schematischen, geschnittenen Darstellung ein weiteres Ausführungsbeispiel einer Schutzeinrichtung sowie eines Werkzeugs eines erfindungsgemäßen chirurgischen Handgeräts,
- Fig. 4: in einer schematischen, perspektivischen Darstellung ein weiteres Ausführungsbeispiel einer erfindungsgemäßen Schutzeinrichtung,
- Fig. 5: in einer schematischen, perspektivischen Darstellung ein weiteres Ausführungsbeispiel einer erfindungsgemäßen Schutzeinrichtung,
- Fig. 6: in einer schematischen, perspektivischen Darstellung ein weiteres Ausführungsbeispiel einer erfindungsgemäßen Schutzeinrichtung,
- Fig. 7: in einer schematischen, perspektivischen Darstellung ein Ausführungsbeispiel des Werkzeugs einer erfindungsgemäßen Vorrichtung,
- Fig. 8: in einer schematischen Darstellung ein weiteres Ausführungsbeispiel des Querschnitts des distalen Rands eines Werkzeugs einer erfindungsgemäßen Vorrichtung,
- Fig. 9: in einer schematischen Darstellung ein weiteres Ausführungsbeispiel des Querschnitts des distalen Rands eines Werkzeugs einer erfindungsgemäßen Vorrichtung,
- Fig. 10: in einer schematischen Darstellung ein weiteres Ausführungsbeispiel einer erfindungsgemäßen Schutzeinrichtung nebst Werkzeug,
- Fig. 11: in einer schematischen Explosivdarstellung das Ausführungsbeispiel aus Figur 10,
- Fig. 12: in einer weiteren schematischen Explosivdarstellung das Ausführungsbeispiel aus Figur 10,
- Fig. 13: in einer schematischen Darstellung ein weiteres Ausführungsbeispiel einer erfindungsgemäßen Schutzeinrichtung nebst Werkzeug und
- Fig. 14: in einer schematischen Explosivdarstellung das Ausführungsbeispiel aus Figur 13.

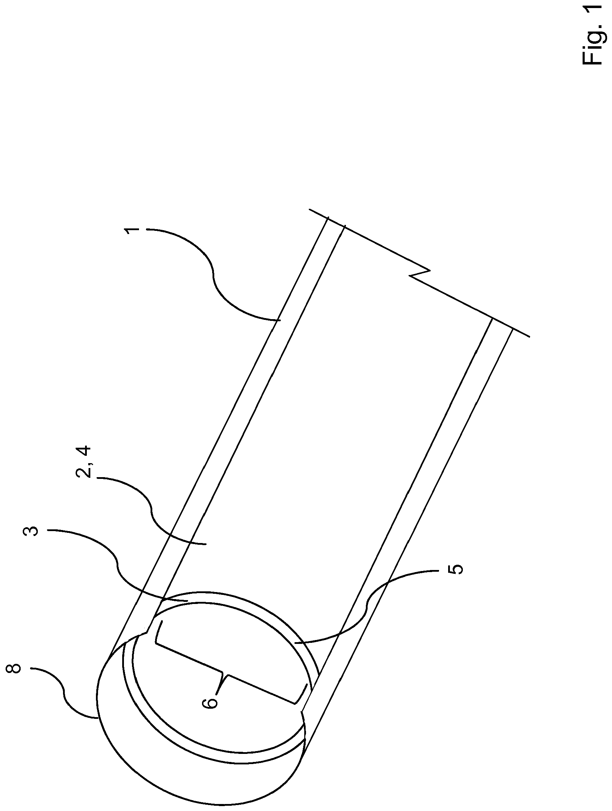

Fig. 1 zeigt in einer schematischen, perspektivischen Darstellung ein Ausführungsbeispiel einer Schutzeinrichtung 1 sowie eines Werkzeugs 2 eines erfindungsgemäßen chirurgischen Handgeräts. Zur Vereinfachung der Darstellung ist in Fig. 1 sowie den nachfolgenden Figuren auf die Darstellung des Handgeräts, d. h. des Winkelstücks bzw. Handstücks, an dem die Schutzeinrichtung 1 und das Werkzeug 2 festgelegt sind, nicht gezeigt.

Aus Fig. 1 ist ersichtlich, dass das Werkzeug 2 als Hohlzylinder ausgebildet ist, wobei an dem distalen Rand 3 des Kopfes 4 ein Wirkbereich 5 zur spanenden Bearbeitung von Knochen angeordnet ist. Im hier dargestellten Ausführungsbeispiel ist der distale Rand 3 flach ausgebildet und weist eine raue Oberfläche auf, nämlich eine sogenannte Diamantierung. Die Schutzeinrichtung 1 umgibt den distalen Rand 3 des Werkzeugs 2 teilweise und erstreckt sich in Axialrichtung teilweise über den distalen Rand 3 hinweg. Dadurch ist lediglich ein Kreisbogen 6 von der Schutzeinrichtung 1 freigegeben, so dass dieser als Wirkbereich 5 zur Bearbeitung des Knochens dient. Des Weiteren geht aus Fig. 1 hervor, dass die Schutzeinrichtung 1 und das Werkzeug 2 bzw. der Kopf 4 des Werkzeugs 2 zylinderförmig ausgebildet sind. Ferner kann die Schutzeinrichtung 1 in Umgangsrichtung nebeneinander angeordnete, beispielsweise ovale, Öffnungen aufweisen. Diese sind zur Vereinfachung in Fig. 1 und den nachfolgenden Figuren nicht dargestellt.

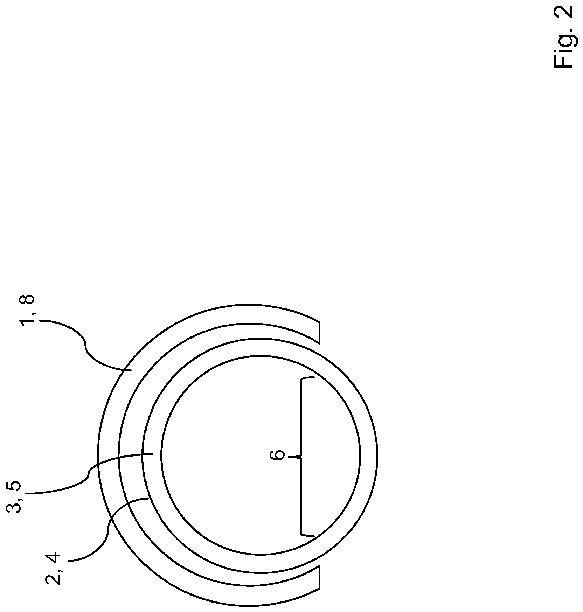

Fig. 2 zeigt in einer schematischen, frontalen Darstellung das Ausführungsbeispiel gemäß Fig. 1. Dabei ist nochmals deutlich zu erkennen, dass die Schutzeinrichtung 1 den Kopf 4 des Werkzeugs 2 derart umgibt, dass lediglich ein Kreisbogen 6 des distalen Randes 3 als Wirkbereich 5 dient. Mit diesem Kreisbogen 6 kann der Operateur ein etwa halbmondförmiges Knochenstück aus dem Knochen herausschälen.

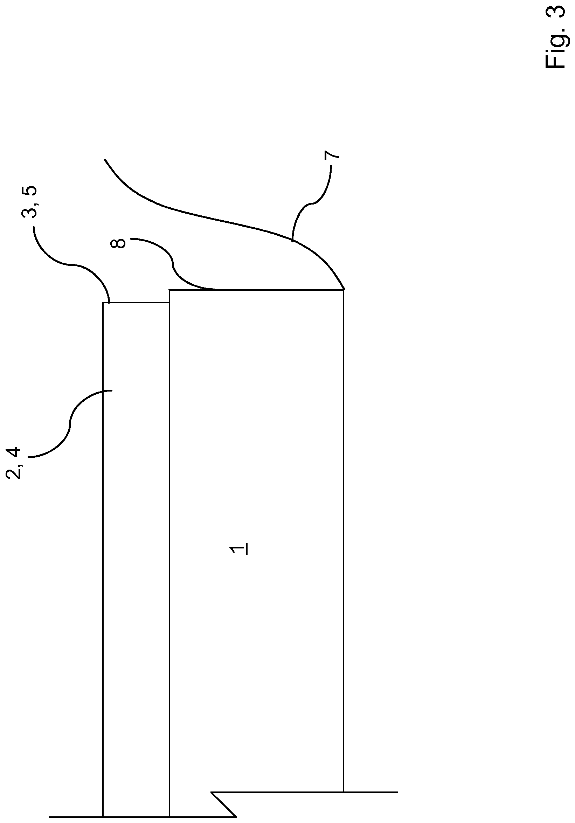

Fig. 3 zeigt in einer schematischen, geschnittenen Darstellung ein weiteres Ausführungsbeispiel einer Schutzeinrichtung 1 sowie eines Werkzeugs 2 eines erfindungs-gemäßen chirurgischen Handgeräts. Das Ausführungsbeispiel gemäß Fig. 3 entspricht dem Ausführungsbeispiel gemäß den Fig. 1 und 2, wobei zusätzlich ein sich in Axialrichtung erstreckendes Führungselement 7 angeordnet ist. Das Führungselement 7 dient zur leichteren Einführung in die Tasche zwischen Weichgewebe und Knochen. Dabei kann das Führungselement 7 aus dem gleichen Material wie die Schutzeinrichtung hergestellt und insbesondere als integraler Bestandteil der Schutzeinrichtung 1 ausgebildet sein.

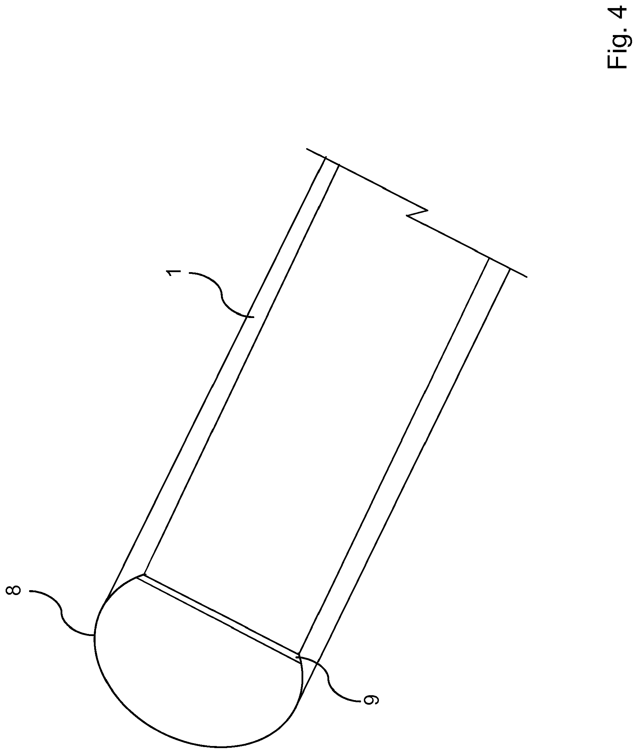

Fig. 4 zeigt in einer schematischen, perspektivischen Darstellung ein weiteres Ausführungsbeispiel einer erfindungsgemäßen Schutzeinrichtung. Zur Vereinfachung der Darstellung ist in Fig. 4 sowie den Fig. 5 und 6 das rotierbare Werkzeug 2 nicht dargestellt. Im Unterschied zu der in den Fig. 1 bis 3 gezeigten Schutzeinrichtung 1 ist an dem freien Ende 8 der Schutzeinrichtung ein radial über das freie Ende 8 hinweg verlaufender Steg 9 ausgebildet. Der Steg 9 dient dazu, ein zu tiefes Eindringen des Werkzeugs 2 in den Knochen zu verhindern.

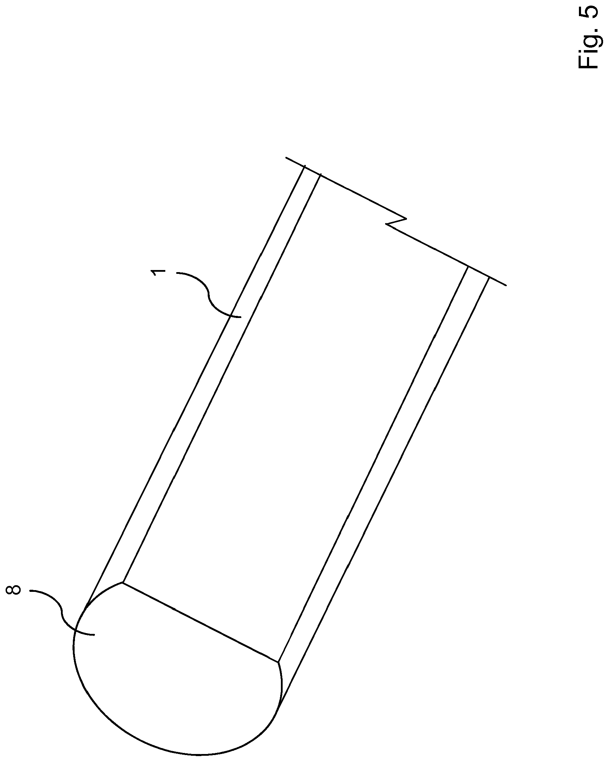

Fig. 5 zeigt in einer schematischen, perspektivischen Darstellung ein weiteres Ausführungsbeispiel einer erfindungsgemäßen Schutzeinrichtung 1. Die Schutzeinrichtung 1 entspricht der Schutzeinrichtung 1 gemäß Fig. 4 wobei an Stelle eines Stegs 9 das freie Ende 8 der Schutzeinrichtung 1 komplett verschlossen ist.

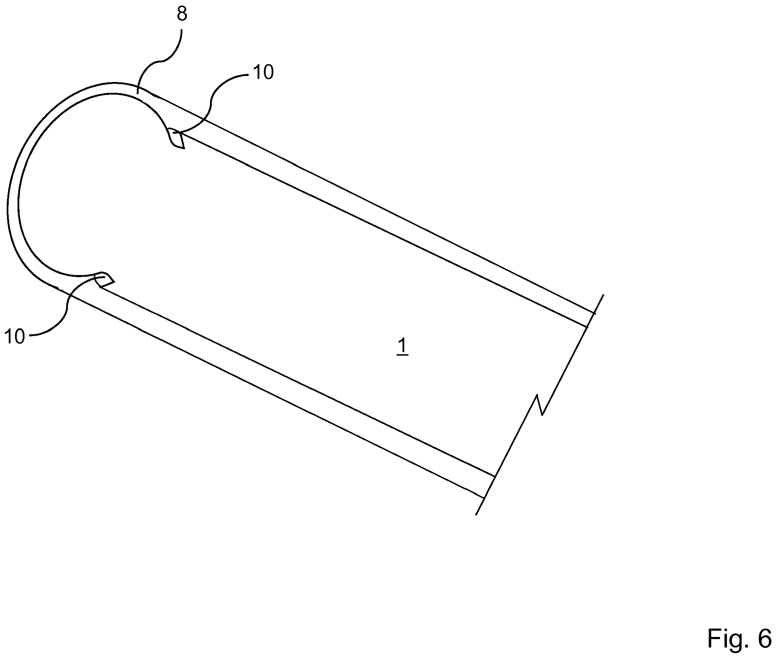

Fig. 6 zeigt in einer schematischen, perspektivischen Darstellung ein weiteres Ausführungsbeispiel einer erfindungsgemäßen Schutzeinrichtung 1. An dem freien Ende 8 der Schutzeinrichtung 1 ist ein sich radial nach innen erstreckender Vorsprung 10 ausgebildet. Der Vorsprung 10 hat in diesem Ausführungsbeispiel die Form eines Kragens, erstreckt sich nämlich nicht nur radial nach innen, sondern auch axial in Richtung des Werkzeugs 2, so dass der distale Rand 3 des Kopfes 2 von dem Vorsprung 10 umschlossen ist. Durch diese konstruktive Maßnahme ist der Kopf 4 des Werkzeugs 2 sowohl radial als auch axial bzw. in Umfangsrichtung umschlossen und somit das umgebende Weichgebe vor Verletzungen geschützt. Dabei sei ausdrücklich darauf hingewiesen, dass der Vorsprung 10 auch derart ausgebildet sein kann, dass er sich lediglich radial nach innen erstreckt und somit den distalen Rand 3 des Werkzeugs 2 in Axialrichtung abdeckt.

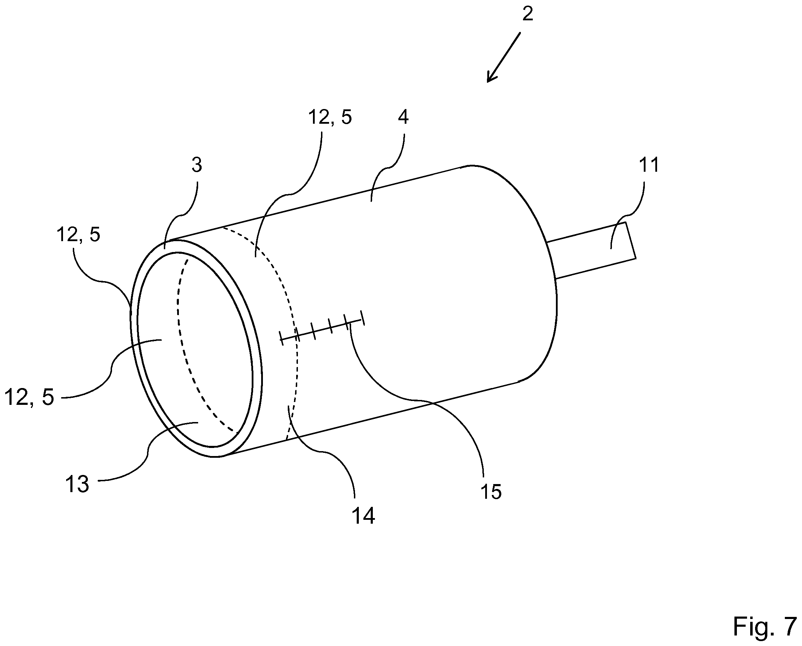

Fig. 7 zeigt in einer schematischen, perspektivischen Darstellung ein Ausführungsbeispiel des Werkzeugs 2 einer erfindungsgemäßen Vorrichtung. Das Werkzeug 2 umfasst einen Anschlussbereich 11 zum Verbinden mit einem hier nicht dargestellten Handgerät. Bei dem Handgerät kann es sich beispielsweise um ein gängiges Winkelstück oder Handstück handeln, welches das Werkzeug 2 in Rotation versetzt.

Das Werkzeug 2 weist des Weiteren einen hohlzylinderförmigen Kopf 4 auf. An dem distalen Rand 3 des Kopfes 4 ist eine raue Oberfläche 12 ausgebildet, die einen Teil des Wirkbereichs 5 bildet. Im hier dargestellten Ausführungsbeispiel ist an der inneren Wandung 13 und der äußeren Wandung 14 eine raue Oberfläche 12 - beispielsweise eine Beschichtung mit Diamantkörner oder Korundkörnern - ausgebildet. Die Ausdehnung der rauen Oberfläche 12 auf der inneren Wandung 13 und der äußeren Wandung 14 ist jeweils durch eine gestrichelte Linie dargestellt. Jedoch ist denkbar, dass die gesamte innere Wandung 13 und/oder äußere Wandung 14 mit einer rauen Oberfläche 12 ausgebildet ist. Der Wirkbereich 5 ist insgesamt durch die raue Oberfläche 12 an dem distalen Rand 3, der inneren Wandung 13 und der äußeren Wandung 14 gebildet.

Auf der äußeren Wandung 14 ist des Weiteren eine Markierung 15 zur Visualisierung der Eindringtiefe des Kopfes 4 in den Knochen ausgebildet.

Im Ausführungsbeispiel gemäß Fig. 7 ist der distale Rand 3 flach ausgebildet, d. h. weist einen flachen bzw. geraden Querschnitt auf.

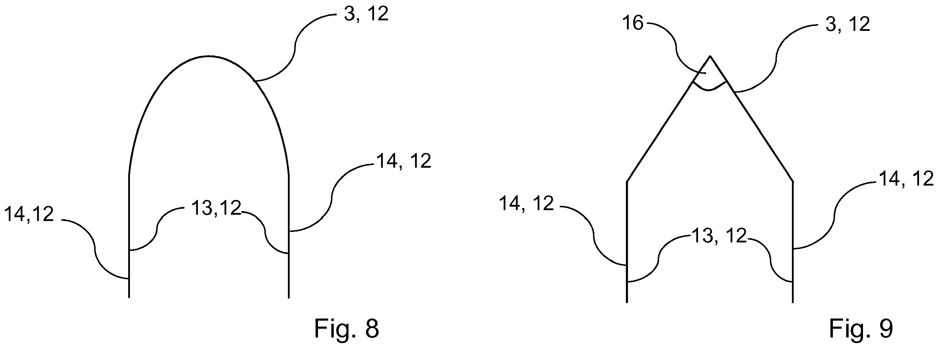

Fig. 8 zeigt in einer schematischen Darstellung ein weiteres Ausführungsbeispiel des Querschnitts des distalen Rands 3 eines Werkzeugs 2 einer erfindungsgemäßen Vorrichtung. Der distale Rand 3 hat gemäß Fig. 8 einen konvexen, nämlich ovalen Querschnitt. Diese Konstruktion zeichnet sich durch ein besonders leichtes Eindringen des distalen Rands 3 in den Knochen aus. Des Weiteren sind in Fig. 8 sowohl die innere Wandung 13 als auch die äußere Wandung 14 und der distale Rand 3 mit einer rauen Oberfläche 12 versehen. Jedoch kann lediglich der distale Rand 3 oder der distale Rand 3 und die innere Wandung 13 oder der distale Rand 3 und die äußere Wandung 14 mit einer rauen Oberfläche 12 ausgebildet sein.

Fig. 9 zeigt in einer schematischen Darstellung ein weiteres Ausführungsbeispiel des Querschnitts des distalen Rands 3 eines Werkzeugs 2 einer erfindungsgemäßen Vorrichtung. Im Gegensatz zu den in den Fig. 7 und Fig. 8 dargestellten Ausführungsbeispielen ist der Querschnitt des distalen Rands 3 in Fig. 9 eckig ausgebildet, weist nämlich einen Winkel 16 auf. An dieser Stelle sei darauf hingewiesen, dass der Übergang zwischen dem distalen Rand 3 und der inneren Wandung 13 bzw. der äußeren Wandung 14 wie in Fig. 9 dargestellt eckig ausgestaltet sein kann. Des Weiteren kann dieser Übergang auch abgerundet realisiert sein. Des Weiteren sind in Fig. 9 sowohl die innere Wandung 13 als auch die äußere Wandung 14 und der distale Rand 3 mit einer rauen Oberfläche 12 versehen. Jedoch kann lediglich der distale Rand 3 oder der distale Rand 3 und die innere Wandung 13 oder der distale Rand 3 und die äußere Wandung 14 mit einer rauen Oberfläche 12 ausgebildet sein.

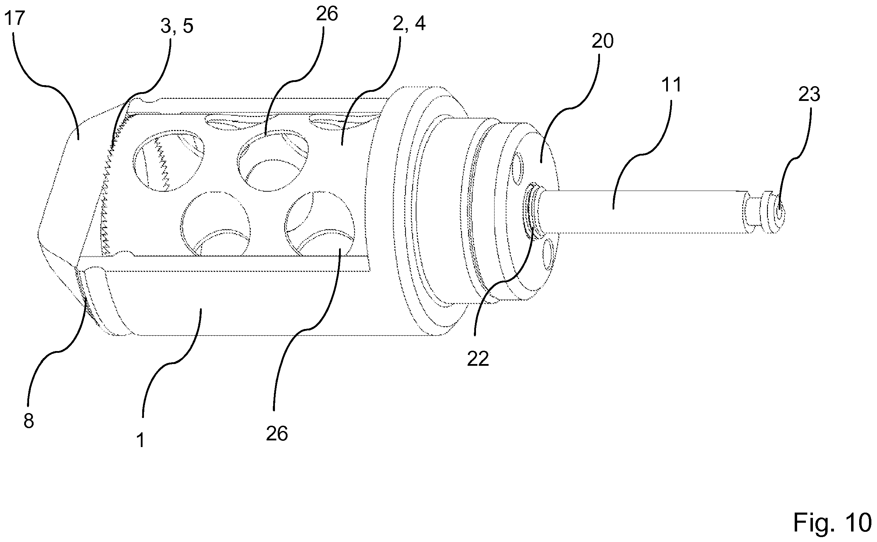

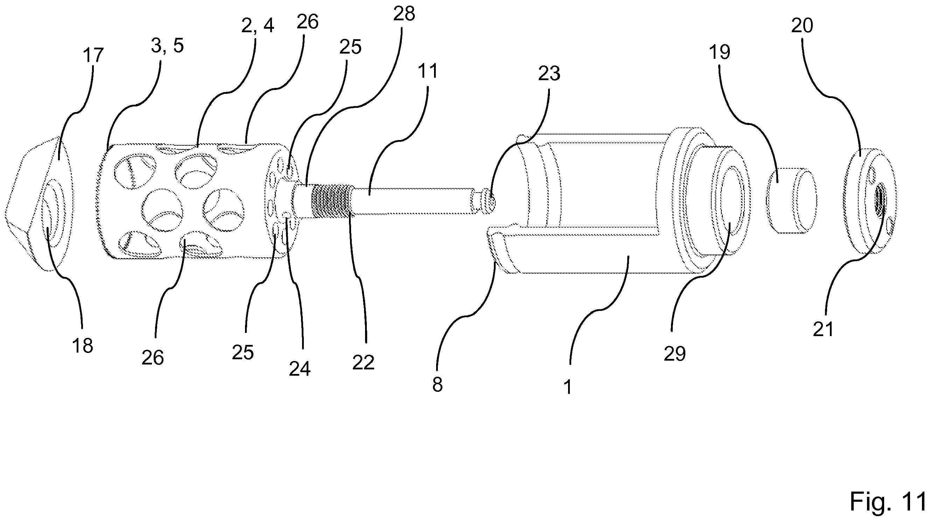

Die Figuren 10 bis 12 zeigen ein weiteres Ausführungsbeispiel einer erfindungsgemäßen Schutzeinrichtung 1 für ein Werkzeug 2. In dem hier dargestellten Ausführungsbeispiel weist der distale Rand 3 des Kopfes 4 Zähne, insbesondere Sägezähne bzw. Schneidzähne, auf, die als Wirkbereich 5 zur spanenden Bearbeitung von Knochen dienen. Dabei sind auch andere Ausgestaltungen des distalen Randes 3 denkbar, beispielsweise entsprechend den Figuren 7 bis 9. Weiterhin können die in den Figuren 1 bis 9 dargestellten Ausführungsbeispiele ein Werkzeug 2 umfassen, das entsprechende Zähne, insbesondere Sägezähne bzw. Schneidzähne, aufweist.

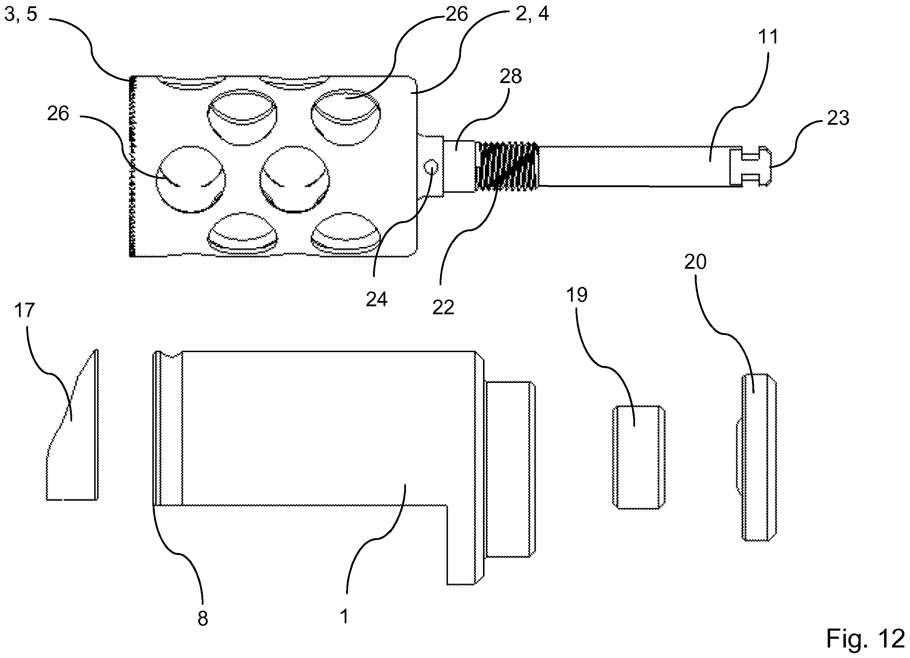

Die Schutzeinrichtung 1 ist im Wesentlichen zylinderförmig bzw. zylindersegmentförmig ausgebildet und weist an dem freien Ende 8 ein Verdrängerelement 17 auf. Das Verdrängerelement 17 dient dazu, die Knochenhaut während des Eingriffs wegzuschieben, so dass das Werkzeug 2 möglichst einfach in den Knochen eindringen kann. Insbesondere aus Fig. 12 geht deutlich hervor, dass die Dicke des Verdrängerelementes 17 von der Außenseite zu der Mitte hin zunächst zunimmt und sodann über einen Abschnitt hinweg konstant bleibt, wodurch die Knochenhaut äußerst effektiv zurückdrängbar ist.

Ein weiterer Vorteil des Verdrängerelements 17 liegt darin, dass dieses den distalen Rand 3 des Werkzeugs 2 nochmals abschirmt, so dass das umliegende Gewebe bestmöglich vor Verletzungen geschützt ist. An dieser Stelle sei ausdrücklich darauf hingewiesen, dass das Verdrängerelement 17 nicht zwangsweise angeordnet sein muss, die Schutzeinrichtung 1 als solche den Kopf 4 nämlich derart umgibt, dass in erfindungsgemäßer Weise lediglich ein Kreisbogen 6 als Wirkbereich 5 dient. An dem Verdrängerelement 17 kann eine Öffnung 18 ausgebildet sein, durch die ein gewonnenes Knochenstück aus dem Inneren der Vorrichtung äußerst einfach entnehmbar ist.

In den Figuren 10 bis 12 ist des Weiteren das Lager 19, beispielsweise ein Kugellager, gezeigt, über welches die Schutzeinrichtung 1 mit dem Werkzeug 2, insbesondere dem Lagersitz 28 des Anschlussbereichs 11 bzw. Schafts, verbunden ist. Ferner kann in der Schutzeinrichtung 1 eine Lageraufnahme 29 ausgebildet sein. Um eine sichere Verbindung der Schutzeinrichtung 1 mit dem Werkzeug 2, insbesondere in Axialrichtung, zu gewährleisten ist ein Haltemittel 20 - bspw. eine Mutter - angeordnet, das mittels eines Innengewindes 21 mit dem Außengewinde 22 des Anschlussbereichs 11 verschraubbar ist.

Dadurch kann das Werkzeug 2 gegenüber der Schutzeinrichtung 1 drehen, so dass die Schutzeinrichtung 1 während des Eingriffs im Wesentlichen drehfest angeordnet ist und das umliegende Gewebe in idealer Weise vor Verletzungen schützt. Die Verbindung der Schutzeinrichtung 1 mit dem nicht dargestellten chirurgischen Handgerät erfolgt somit über den Schaft bzw. Anschlussbereich 11 des Werkzeugs 2, welcher als Verbindungselement dient. Des Weiteren ist denkbar, dass die Schutzeinrichtung 1 alternativ oder zusätzlich über ein weiteres - nicht dargestelltes - Verbindungselement mit dem chirurgischen Handgerät verbunden ist. Ein solches weiteres Verbindungselement hat den zusätzlichen Effekt, dass die Schutzeinrichtung 1 drehfest an dem chirurgischen Handgerät angeordnet ist, so dass ein "Mitdrehen" mit dem Werkzeug 2 vermieden wird.

An dem proximalen Ende des Anschlussbereichs 11 ist ein Einlass 23 für eine Kühlflüssigkeit ausgebildet, die in Strömungsverbindung mit dem Auslass 24 steht. Somit kann Kühlflüssigkeit von dem Handgerät über den Einlass 23 zu dem Auslass 24 gefördert werden, wobei die Kühlflüssigkeit über die ersten Kühlöffnungen 25 des Werkzeugs 2 in das Innere der Schutzeinrichtung 1 einbringbar ist. Des Weiteren sind zweite Kühlöffnungen 26 an dem Werkzeug 2 ausgebildet, durch welche die Oberfläche des Werkzeugs 2 und somit die Reibung mit der Eingriffstelle minimiert wird. Durch diese Maßnahmen wird eine Überhitzung des zu bearbeitenden Gewebes bzw. Knochens vermieden. An dieser Stelle sei darauf hingewiesen, dass zur besseren Übersicht lediglich einige der ersten Kühlöffnungen 25 und der zweiten Kühlöffnungen 26 mit einem Bezugszeichen versehen sind.

Wie Fig. 12 zu entnehmen ist, ist das distale Ende der Schutzeinrichtung 1, nämlich das Verdrängerelement 17 als separates Bauteil ausgebildet. Das Verdrängerelement 17 kann bspw. mittels eines Klebstoffs mit dem freien Ende 8 der Schutzeinrichtung 1 verbunden sein. In vorteilhafter Weise kann der Klebstoff derart ausgewählt, bzw. eingestellt sein, dass er bei einem Wiederaufbereiten bzw. Sterilisieren, insbesondere Autoklavieren, der Schutzeinrichtung 1 schmilzt, so dass die Schutzeinrichtung 1 nicht mehr verwendbar ist. Alternativ und/oder zusätzlich kann die Schutzeinrichtung zumindest teilweise aus einem Material bestehen, das seine Form und/oder Farbe bei einem Wiederaufbereiten ändert.

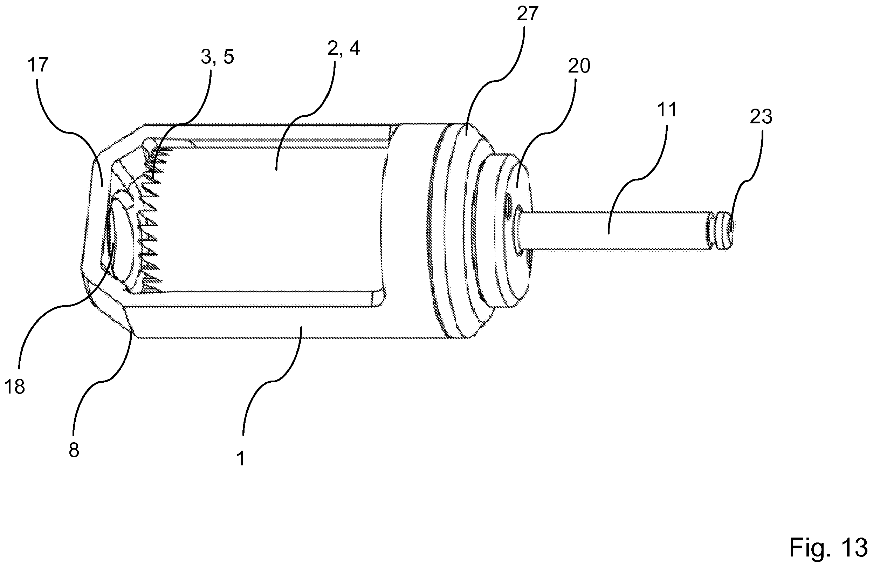

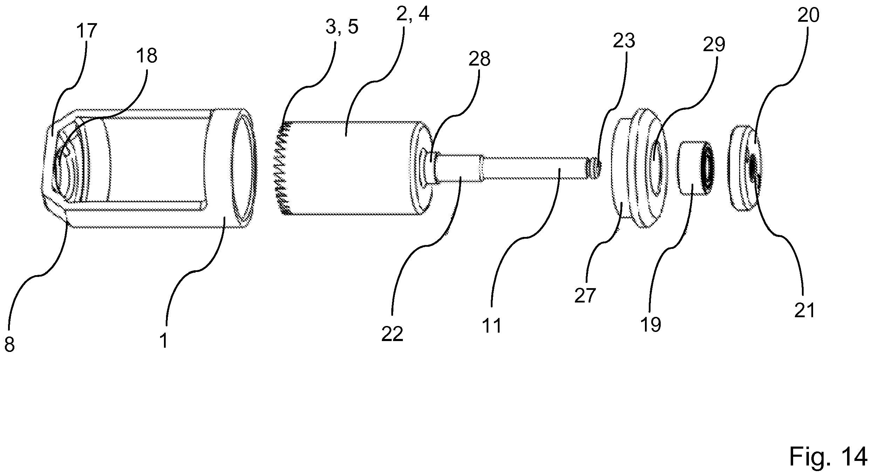

In den Figuren 13 und 14 ist ein weiteres Ausführungsbeispiel einer erfindungsgemäßen Schutzeinrichtung 1 nebst Werkzeug 2 dargestellt. Dabei ist das Verdrängerelement 17 ein integraler Bestandteil der Schutzeinrichtung 1, d.h. einteilig mit dieser ausgebildet. Des Weiteren ist das proximale Ende 27 der Schutzeinrichtung 1 als separates Bauteil ausgestaltet und bspw. über einen Klebstoff mit der Schutzeinrichtung 1 verbunden. In vorteilhafter Weise kann der Klebstoff derart ausgewählt, bzw. eingestellt sein, dass er bei einem Wiederaufbereiten bzw. Sterilisieren, insbesondere Autoklavieren, der Schutzeinrichtung 1 schmilzt, so dass die Schutzeinrichtung 1 nicht mehr verwendbar ist.

Das Ausführungsbeispiel gemäß den Figuren 13 und 14 entspricht des Weiteren dem in den Figuren 10 bis 12 dargestellten Ausführungsbeispiel. Insbesondere kann das in den Figuren 13 und 14 dargestellte Werkzeug 2 erste Kühlöffnungen und zweite Kühlöffnungen aufweisen, wie dies in den Figuren 10 bis 12 dargestellt ist. Zur Vermeidung von Wiederholungen wird an dieser Stelle auf die voranstehende Beschreibung der Figuren 10 bis 12 verwiesen, die analog für das Ausführungsbeispiel der Figuren 13 und 14 gilt.

Hinsichtlich weiterer vorteilhafter Ausgestaltungen der erfindungsgemäßen Vorrichtung wird zur Vermeidung von Wiederholungen auf den allgemeinen Teil der Beschreibung sowie auf die beigefügten Ansprüche verwiesen. Schließlich sei ausdrücklich darauf hingewiesen, dass die voranstehend beschriebenen Ausführungsbeispiele der erfindungsgemäßen Vorrichtung lediglich zur Erörterung der beanspruchten Lehre dienen, diese jedoch nicht auf die Ausführungsbeispiele einschränken.

### Bezugszeichenliste

- 1: Schutzeinrichtung
- 2: Werkzeug
- 3: distale Rand
- 4: Kopf
- 5: Wirkbereich
- 6: Kreisbogen
- 7: Führungselement
- 8: freies Ende
- 9: Steg
- 10: Vorsprung
- 11: Anschlussbereich
- 12: raue Oberfläche
- 13: innere Wandung
- 14: äußere Wandung
- 15: Markierung
- 16: Winkel
- 17: Verdrängerelement
- 18: Öffnung
- 19: Lager
- 20: Haltemittel
- 21: Innengewinde
- 22: Außengewinde
- 23: Einlass
- 24: Auslass
- 25: erste Kühlöffnungen
- 26: zweite Kühlöffnungen
- 27: proximale Ende
- 28: Lagersitz
- 29: Lageraufnahme

## Patentansprüche

1. Chirurgisches Handgerät, nämlich Handstück oder Winkelstück zur Anwendung in der Oralchirurgie, mit einem rotierbaren Werkzeug (2), wobei ein Kopf (4) des Werkzeugs (2) als Hohlzylinder ausgebildet ist und wobei an einem distalen Rand (3) des Kopfes (4) ein Wirkbereich (5), insbesondere zur spanenden Bearbeitung von Knochen, angeordnet ist,
**dadurch gekennzeichnet, dass** eine Schutzeinrichtung (1) angeordnet ist, die den distalen Rand (3) des Werkzeugs (2) teilweise umgibt und sich in Axialrichtung teilweise über den distalen Rand (3) hinweg erstreckt, so dass lediglich ein Kreisbogen (6) des distalen Randes (3) als Wirkbereich (5) dient.

2. Chirurgisches Handgerät nach Anspruch 1, **dadurch gekennzeichnet, dass** die Schutzeinrichtung (1) den Kopf (4) derart umgibt, dass ein von dem Kreisbogen (6) gebildetes Kreissegment eine Segmenthöhe von 2,5 mm bis 3,5 mm, insbesondere 3,1 mm bis 3,4 mm, vorzugsweise 3,2 mm, aufweist.

3. Chirurgisches Handgerät nach Anspruch 1 oder 2, **dadurch gekennzeichnet, dass** die Schutzeinrichtung (1) und/oder das Werkzeug (2) lösbar mit dem Handgerät verbunden ist/sind.

4. Chirurgisches Handgerät nach einem der Ansprüche 1 bis 3, **dadurch gekennzeichnet, dass** die Schutzeinrichtung (1) drehbar mit dem Werkzeug (2) gekoppelt ist.

5. Chirurgisches Handgerät nach Anspruch 4, **dadurch gekennzeichnet, dass** die Schutzeinrichtung (1) über ein Lager (19), insbesondere ein Wälzlager bzw. Kugellager bzw. Rillenkugellager, mit dem Werkzeug (2) gekoppelt ist.

6. Chirurgisches Handgerät nach einem der Ansprüche 1 bis 5, **dadurch gekennzeichnet, dass** die Schutzeinrichtung (1) zumindest bereichsweise zylinderförmig ausgebildet ist.

7. Chirurgisches Handgerät nach einem der Ansprüche 1 bis 6, **dadurch gekennzeichnet, dass** an dem freien Ende (8) der Schutzeinrichtung (1) ein sich radial nach innen erstreckender Vorsprung (10) ausgebildet ist.

8. Chirurgisches Handgerät nach einem der Ansprüche 1 bis 7, **dadurch gekennzeichnet, dass** an dem freien Ende (8) der Schutzeinrichtung (1) ein radial über das freie Ende (8) verlaufender, vorzugsweiser runder, Steg (9) ausgebildet ist.

9. Chirurgisches Handgerät nach einem der Ansprüche 1 bis 8, **dadurch gekennzeichnet, dass** an der Schutzeinrichtung (1) zumindest eine Austrittsöffnung angeordnet ist, wobei die Austrittsöffnung in Strömungsverbindung mit einem in der Schutzeinrichtung (1) verlaufenden Kanal steht, um das Werkzeug (2) mit einem Kühlmedium zu beaufschlagen.

10. Chirurgisches Handgerät nach einem der Ansprüche 1 bis 9, **dadurch gekennzeichnet, dass** die Schutzeinrichtung (1) in Umfangsrichtung nebeneinander angeordnete, vorzugsweise ovale, Öffnungen aufweist.

11. Chirurgisches Handgerät nach einem der Ansprüche 1 bis 10, **dadurch gekennzeichnet, dass** an der Schutzeinrichtung (1) ein sich in Axialrichtung erstreckendes Führungselement (7) ausgebildet ist.

12. Kombination aus einem rotierbaren Werkzeug (2) und einer Schutzeinrichtung (1), zur Anwendung in der Oralchirurgie, wobei das Werkzeug (2) einen mit einem chirurgischen Handgerät verbindbaren Anschlussbereich (11) aufweist, der vorgesehen ist, für die Schutzeinrichtung (1) als Verbindungselement zur lösbaren Befestigung an dem chirurgischen Handgerät zu dienen, wobei die Schutzeinrichtung (1) drehbar mit dem Werkzeug (2) gekoppelt ist und von dem Werkzeug (2) getragen wird, wobei ein Kopf (4) des Werkzeugs (2) hohlzylinderförmig ausgebildet ist, wobei die Schutzeinrichtung (1) einen distalen Rand (3) des Werkzeugs (2) teilweise umgibt und sich in Axialrichtung teilweise über den distalen Rand (3) hinweg erstreckt, so dass lediglich ein Kreisbogen (6) des distalen Randes (3) als Wirkbereich (5), insbesondere zur spanenden Bearbeitung von Knochen, dient.

13. Schutzeinrichtung (1) nach Anspruch 12, **dadurch gekennzeichnet, dass** die Schutzeinrichtung (1) über ein Lager (19), insbesondere ein Wälzlager bzw. ein Kugellager bzw. ein Rillenkugellager, mit dem Werkzeug (2) gekoppelt ist.

## Claims

1. Surgical hand-held device, that is to say, handpiece or angle piece for use in oral surgery, having a rotating tool (2), wherein a head (4) of the tool (2) is constructed as a hollow cylinder and wherein at a distal edge (3) of the head (4) an active region (5), in particular for cutting processing of bones, is arranged,
**characterised in that** there is arranged a protection device (1) which partially surrounds the distal edge (3) of the tool (2) and which extends in an axial direction partially over the distal edge (3) so that only a circular arc (6) of the distal edge (3) acts as an active region (5).

2. Surgical hand-held device according to claim 1, **characterised in that** the protection device (1) surrounds the head (4) in such a manner that a circle segment formed by the circular arc (6) has a segment height of from 2.5 mm to 3.5 mm, in particular from 3.1 mm to 3.4 mm, preferably 3.2 mm.

3. Surgical hand-held device according to claim 1 or 2, **characterised in that** the protection device (1) and/or the tool (2) is/are releasably connected to the hand-held device.

4. Surgical hand-held device according to any one of claims 1 to 3, **characterised in that** the protection device (1) is rotatably coupled to the tool (2).

5. Surgical hand-held device according to claim 4, **characterised in that** the protection device (1) is coupled to the tool (2) by means of a bearing (19), in particular a roller bearing or ball bearing or deep groove ball bearing.

6. Surgical hand-held device according to any one of claims 1 to 5, **characterised in that** the protection device (1) is constructed at least partially in a cylindrical manner.

7. Surgical hand-held device according to any one of claims 1 to 6, **characterised in that** at the free end (8) of the protection device (1) a projection (10) which extends radially inwards is formed.

8. Surgical hand-held device according to any one of claims 1 to 7, **characterised in that** a preferably round web (9) which extends radially over the free end (8) is formed at the free end (8) of the protection device (1).

9. Surgical hand-held device according to any one of claims 1 to 8, **characterised in that** at least one discharge opening is arranged in the protection device (1), wherein the discharge opening is connected in terms of flow to a channel which extends in the protection device (1) in order to act on the tool (2) with a cooling medium.

10. Surgical hand-held device according to any one of claims 1 to 9, **characterised in that** the protection device (1) has preferably oval openings which are arranged beside each other in the peripheral direction.

11. Surgical hand-held device according to any one of claims 1 to 10, **characterised in that** a guide element (7) which extends in an axial direction is formed on the protection device (1).

12. Combination of a rotating tool (2) and a protection device (1) for use in oral surgery, wherein the tool (2) has a connection region (11) which can be connected to a surgical hand-held device and which is provided for the protection device (1) to act as a connection element for releasably securing to the surgical hand-held device, wherein the protection device (1) is rotatably coupled to the tool (2) and is carried by the tool (2), wherein a head (4) of the tool (2) is constructed in a hollow-cylindrical manner, wherein the protection device (1) partially surrounds a distal edge (3) of the tool (2) and extends in an axial direction partially over the distal edge (3) so that only a circular arc (6) of the distal edge (3) acts as an active region (5), in particular for cutting processing of bones.

13. Protection device (1) according to claim 12, **characterised in that** the protection device (1) is coupled to the tool (2) by means of a bearing (19), in particular a roller bearing or a ball bearing or a deep-groove ball bearing.

## Revendications

1. Appareil chirurgical portatif, à savoir une pièce à main ou une pièce angulaire, destiné à être utilisé en chirurgie orale, avec un outil rotatif (2), une tête (4) de l'outil (2) étant conçue comme un cylindre creux et une zone active (5) étant disposée sur un bord distal (3) de la tête (4), plus particulièrement pour un usinage d'os avec enlèvement de copeaux,
**caractérisé en ce qu'**un dispositif de protection (1) est disposé, qui entoure partiellement le bord distal (3) de l'outil (2) et s'étend dans la direction axiale partiellement sur le bord radial (3), de façon à ce qu'un seul arc de cercle (6) du bord distal (3) sert de zone active (5).

2. Appareil chirurgical portatif selon la revendication 1, **caractérisé en ce que** le dispositif de protection (1) entoure la tête (4) de façon à ce qu'un segment de cercle formé par l'arc de cercle (6) présente une hauteur de segment de 2,5 mm à 3,5 mm, plus particulièrement de 3,1 mm à 3,4 mm, de préférence de 3,2 mm.

3. Appareil chirurgical portatif selon la revendication 1 ou 2, **caractérisé en ce que** le dispositif de protection (1) et/ou l'outil (2) est/sont relié(s) de manière amovible avec l'appareil portatif.

4. Appareil chirurgical portatif selon l'une des revendications 1 à 3, **caractérisé en ce que** le dispositif de protection (1) est couplé de manière rotative avec l'outil (2).

5. Appareil chirurgical portatif selon la revendication 4, **caractérisé en ce que** le dispositif de protection (1) est couplé, par l'intermédiaire d'un palier (19), plus particulièrement un palier à rouleaux ou un palier à billes ou un palier à billes rainurées, avec l'outil (2).

6. Appareil chirurgical portatif selon l'une des revendications 1 à 5, **caractérisé en ce que** le dispositif de protection (1) est réalisé, au moins à certains endroits, sous une forme cylindrique.

7. Appareil chirurgical portatif selon l'une des revendications 1 à 6, **caractérisé en ce que**, au niveau de l'extrémité libre (8) du dispositif de protection (1), est réalisée une saillie (10) s'étendant radialement vers l'intérieur.

8. Appareil chirurgical portatif selon l'une des revendications 1 à 7, **caractérisé en ce que**, au niveau de l'extrémité libre (8) du dispositif de protection (1), est réalisée une nervure (9) s'étendant radialement au-dessus de l'extrémité libre (8), de préférence ronde.

9. Appareil chirurgical portatif selon l'une des revendications 1 à 8, **caractérisé en ce que**, au niveau du dispositif de protection (1) est disposée au moins une ouverture de sortie, l'ouverture de sortie étant en liaison d'écoulement avec un canal s'étendant dans le dispositif de protection (1), afin d'alimenter l'outil (2) avec un fluide de refroidissement.

10. Appareil chirurgical portatif selon l'une des revendications 1 à 9, **caractérisé en ce que** le dispositif de protection (1) comprend, sur sa circonférence, des ouvertures juxtaposées, de préférence ovales.

11. Appareil chirurgical portatif selon l'une des revendications 1 à 9, **caractérisé en ce que**, au niveau du dispositif de protection (1) est prévu un élément de guidage (7) s'étendant dans la direction axiale.

12. Combinaison d'un outil rotatif (2) et d'un dispositif de protection (1), destinée à être utilisée en chirurgie orale, l'outil (2) comprenant une zone de raccordement (11) pouvant être reliée avec un appareil chirurgical portatif, qui sert, pour le dispositif de protection (1), d'élément de liaison pour une fixation amovible à l'appareil chirurgical portatif, le dispositif de protection (1) étant couplé de manière rotative avec l'outil (2) et étant supporté par l'outil (2), une tête (4) de l'outil (2) présentant la forme d'un cylindre creux, le dispositif de protection (1) entourant partiellement un bord distal (3) de l'outil (2) et s'étendant dans la direction axiale partiellement sur le bord distal (3), de façon à ce que seul un arc de cercle (6) du bord distal (3) serve de zone active (5), plus particulièrement pour l'usinage d'os avec enlèvement de copeaux.

13. Dispositif de protection (1) selon la revendication 12, **caractérisé en ce que** le dispositif de protection (1) est couplé avec l'outil (2) par l'intermédiaire d'un palier (19), plus particulièrement un palier à rouleaux ou un palier à billes ou un palier à billes rainurées.
